# EUROPEAN PATENT APPLICATION

(11) **EP 3 892 271 A1**
(43) Date of publication of application: **13.10.2021**
(21) Application number: 19892385.6
(22) Date of filing: 02.12.2019
(51) Int. Cl.: A61K 31/352, A61P 9/00, A23L 33/10, A23L 2/52, A23L 2/38

(54) **BLOOD FLOW IMPROVEMENT COMPOSITION AND VASCULAR ENDOTHELIUM FUNCTION IMPROVEMENT COMPOSITION**

(30) Priority: 06.12.2018 JP 2018229132
(71) Applicant: Suntory Holdings Limited, Osaka 530-8203 (JP)
(72) Inventor: FUKIZAWA, Shinya, Soraku-gun, Kyoto 619-0284 (JP)
(74) Representative: Vossius & Partner Patentanwälte Rechtsanwälte mbB
(86) International application number: PCT/JP2019/046981
(87) International publication number: WO 2020/116381

(57) **Abstract**

The present invention aims to provide a novel composition for improving blood flow, the composition being capable of improving blood flow by improving vascular endothelial function; a novel composition for improving vascular endothelial function, the composition being capable of improving vascular endothelial function; a novel method of improving blood flow; a novel method of improving vascular endothelial function; and the like. The present invention relates to, for example, a composition for improving blood flow, the composition containing isoxanthohumol as an active ingredient.

## Description

### TECHNICAL FIELD

The present invention relates to a composition for improving blood flow and a composition for improving vascular endothelial function. The present invention also relates to a method of improving blood flow, a method for improving vascular endothelial function, and the like.

### BACKGROUND ART

Vascular endothelial cells make up the lining of blood vessels, and are known to have a function to produce physiologically active substances such as nitric oxide (NO). Nitric oxide is one of vascular endothelium-derived relaxing factors, and is produced as a by-product of a reaction *in vivo* in which L-arginine is converted into L-citrulline by NO synthase (NOS). Promoting nitric oxide production in vascular endothelial cells is effective in relaxing and widening blood vessels and thus improving blood flow and the like. Non-Patent Literature 1 suggests that aging-related reduction in vascular relaxation can be explained based on the decrease in NO production from vascular endothelial cells (reduced vascular endothelial function) and the like. Specifically, aging-related reduction in the level of endothelial NO synthase (eNOS) protein expression, and reduced stimulus to activate eNOS associated with decreased female hormone secretion are considered to lead to poor vascular relaxation or reduced blood flow. Patent Literature 1 describes an agent that accelerates the activity of vascular endothelial nitric oxide synthetase, the agent containing a Balinese long pepper extract as an active ingredient.

Smoking, excessive salt intake, and type II diabetes, and the like have been reported to be factors that reduce vascular endothelial function. A sustained condition of reduced vascular endothelial function may lead to progression of arteriosclerosis. Patent Literature 2 discloses a SR-B1 protein expression enhancer containing xanthohumol as an active ingredient. Intake of xanthohumol is expected to improve cholesterol metabolism by enhancing expression of SR-B1 protein that functions as a high-density lipoprotein (HDL) receptor. While it has been reported that improvement in cholesterol metabolism reduces risks of arteriosclerosis, some recent reports suggest a paradoxical hypothesis, as claimed in Non-Patent Literature 2, and the truth is not sufficiently elucidated. Meanwhile, since reduced vascular endothelial function, which is a pre-stage of arteriosclerosis, is reversible, early detection of a condition of reduced vascular endothelial function and intervention to enhance the function are considered to be clinically significant.

Reduced vascular endothelial function is also deeply involved in abnormal physical conditions that are noticed in daily life, such as cold sensitivity, shoulder stiffness, and menopausal disorders among middle-aged women. Effects that can be expected from improved blood flow by improving vascular endothelial function and thus improving vascular relaxation include prevention or reduction of swelling, body temperature maintenance, prevention or reduction of cold sensitivity, warm-feeling (temperature sensation), prevention or reduction of shoulder stiffness, recovery from fatigue, and fatigue reduction. For example, Non-Patent Literature 3 reports that oral intake of L-citrulline, which is an amino acid having an effect of promoting NO production, provided an effect of reducing lower limb swelling. Non-Patent Literature 4 reports that oral intake of glucosyl hesperidin, which is a glycoside of hesperetin having an effect of promoting NO production, provided an effect of reducing cold sensitivity and an effect of improving skin conditions.

### CITATION LIST

### - Patent Literature

Patent Literature 1: JP 2005-298429 A
Patent Literature 2: JP 2011-256133 A

### - Non-Patent Literature

Non-Patent Literature 1: Biomedical Gerontology 38(3); 11-18, 2014
Non-Patent Literature 2: Expert Rev Clin Pharmacol 2015; 8(2): 189-99
Non-Patent Literature 3: Jpn Pharmacol Ther 2012; 40(9): 787-94
Non-Patent Literature 4: Bulletin of Applied Glycoscience Vol. 1, No. 2, 186-193 (2011)

### SUMMARY OF INVENTION

### - Technical Problem

The present invention aims to provide a novel composition for improving blood flow, the composition being capable of improving blood flow by improving vascular endothelial function. The present invention also aims to provide a composition for improving vascular endothelial function, the composition being capable of improving vascular endothelial function. The present invention also aims to provide a method of improving blood flow, a method of improving vascular endothelial function, and the like.

### - Solution to Problem

As a result of intensive studies to solve the above problems, the present inventors found that isoxanthohumol and xanthohumol, which are types of polyphenols, have an effect of promoting nitric oxide production in vascular endothelial cells and exert an effect of improving vascular endothelial function and an effect of improving blood flow.

Specifically, the present invention relates to, but is not limited to, a composition for improving blood flow, a composition for improving vascular endothelial function, and the like described below.
(1) A composition for improving blood flow, the composition containing isoxanthohumol as an active ingredient.
(2) The composition for improving blood flow according to (1) above, wherein the composition improves blood flow by improving vascular endothelial function.
(3) The composition for improving blood flow according to (2) above, wherein the improving vascular endothelial function is promoting nitric oxide production in vascular endothelial cells.
(4) A composition for improving vascular endothelial function, the composition containing isoxanthohumol as an active ingredient.
(5) A composition for improving blood flow which improves blood flow by improving vascular endothelial function, the composition containing xanthohumol as an active ingredient.
(6) The composition for improving blood flow according to (5) above, wherein the improving vascular endothelial function is promoting nitric oxide production in vascular endothelial cells.
(7) A composition for improving vascular endothelial function, the composition containing xanthohumol as an active ingredient.
(8) The composition according to any one of (1) to (7) above, wherein the composition is a food or beverage.
(9) The composition according to any one of (1) to (8) above, wherein the composition is a beverage.
(10) The composition according to (9) above, wherein the beverage is a tea-based beverage, a coffee beverage, an alcoholic beverage, a non-alcoholic beer-taste beverage, a carbonated beverage, a functional beverage, a fruit and/or vegetable-based beverage, a lactic beverage, a soy milk beverage, or flavored water.
(11) The composition according to any one of (1) to (10) above, wherein the composition is labeled with one or more of the following function claims: "improving vascular endothelial function", "preventing a reduction in vascular endothelial function", "maintaining the health of blood vessels", "maintaining blood vessel flexibility", "widening blood vessels to maintain blood flow", "promoting blood circulation", "improving blood flow to maintain the body temperature", "improving blood flow to prevent or reduce cold sensitivity", "warm-feeling (temperature sensation)", "improving blood flow to prevent or reduce shoulder stiffness", "improving blood flow to promote recovery from fatigue", "reducing fatigue", "preventing or reducing swelling", and "improving blood flow to improve skin conditions".
(12) A method of improving blood flow, the method including administering or feeding isoxanthohumol to a subject.
(13) A method of improving vascular endothelial function, the method including administering or feeding isoxanthohumol to a subject.
(14) A method of improving blood flow by improving vascular endothelial function, the method including administering or feeding xanthohumol to a subject.
(15) A method of improving vascular endothelial function, the method including administering or feeding xanthohumol to a subject.
(16) Use of isoxanthohumol for improving blood flow.
(17) Use of isoxanthohumol for improving vascular endothelial function.
(18) Use of xanthohumol for improving blood flow by improving vascular endothelial function.
(19) Use of xanthohumol for improving vascular endothelial function.

### - Advantageous Effects of Invention

The present invention can provide a novel composition for improving blood flow, the composition being capable of improving blood flow by improving vascular endothelial function. The present invention can also provide a composition for improving vascular endothelial function, the composition being capable of improving vascular endothelial function. The present invention can also provide a method of improving blood flow, a method of improving vascular endothelial function, and the like.

### DESCRIPTION OF EMBODIMENTS

A first embodiment of the present invention relates to a composition for improving blood flow, the composition containing isoxanthohumol as an active ingredient. Hereinafter, the composition for improving blood flow according to the first embodiment of the present invention is also referred to as a "first composition for improving blood flow". Isoxanthohumol has an effect of improving vascular endothelial function. In one embodiment, the first composition for improving blood flow of the present invention is suitably used as a composition for improving blood flow which improves blood flow by improving vascular endothelial function.

A second embodiment of the present invention relates to a composition for improving vascular endothelial function, the composition containing isoxanthohumol as an active ingredient. Hereinafter, the composition for improving vascular endothelial function according to the second embodiment of the present invention is also referred to as a "first composition for improving vascular endothelial function".

A third embodiment of the present invention relates to a composition for improving blood flow which improves blood flow by improving vascular endothelial function, the composition containing xanthohumol as an active ingredient. Hereinafter, the composition for improving blood flow according to the third embodiment of the present invention is also referred to as a "second composition for improving blood flow".

A fourth embodiment of the present invention relates to a composition for improving vascular endothelial function, the composition containing xanthohumol as an active ingredient. Hereinafter, the composition for improving vascular endothelial function according to the fourth embodiment of the present invention is also referred to as a "second composition for improving vascular endothelial function".

In the present invention, preferably, the "improving vascular endothelial function" is promoting nitric oxide production in vascular endothelial cells. The improvement in vascular endothelial function can be measured and evaluated non-invasively by flow-mediated dilation (FMD) inspection, plethysmography, or the like. In one embodiment, the first and second compositions for improving vascular endothelial function of the present invention can be used to promote nitric oxide production in vascular endothelial cells. The composition for improving blood flow is suitably used to improve blood flow by improving vascular endothelial function. The "improving blood flow" refers to increasing the blood flow rate, maintaining the blood flow rate, suppressing a decrease in blood flow rate, increasing the amount of blood flow, and the like. The blood flow can be measured using a laser blood flow meter or by a technique such as arterial spin labeling (ASL), fNIRS, or the like.

The first composition for improving blood flow and the first composition for improving vascular endothelial function of the present invention each contain isoxanthohumol as an active ingredient. Isoxanthohumol is a compound formed by isomerization of xanthohumol. Isoxanthohumol is not limited by the production method or anything.

Isoxanthohumol can be prepared, for example, through a process such as heating of a hop *(Humulus lupulus,* a plant in the family Cannabaceae) extract. Heating of a hop extract can produce isoxanthohumol in the extract. A hop extract is usually prepared through a process involving extraction of hop cones with a solvent and purification as needed. A hop extract can be obtained by a known preparation method. Hops can be extracted, for example, by a method that uses an ethanol solvent, which is used as a preparation method of a hop extract for beer brewing. A hop extract is commercially available, and a commercial hop extract can also be used. A hop extract is heated to produce isoxanthohumol preferably at 80°C to 140°C (more preferably 85°C to 100°C) for 15 minutes to 5 hours (more preferably 20 minutes to 3 hours). The hop extract is purified to prepare isoxanthohumol by a known method. Isoxanthohumol can also be produced by isomerizing xanthohumol by heating. Here, the heating temperature is preferably 80°C to 140°C (more preferably 85°C to 100°C) for 15 minutes to 5 hours (more preferably 20 minutes to 3 hours). Isoxanthohumol obtained by isomerization can be concentrated or purified by a known method (e.g., filtration, vacuum concentration, or lyophilization) as needed.

Commercially available isoxanthohumol can also be used. In the present invention, purified isoxanthohumol may be used. The composition of the present invention may contain a material derived from plants rich in isoxanthohumol as long as the effect of the present invention can be achieved. The material derived from plants rich in isoxanthohumol may be a heat-treated hop extract or its concentrate, dried matter, or the like, for example.

The second composition for improving blood flow and the second composition for improving vascular endothelial function of the present invention each contain xanthohumol as an active ingredient. Xanthohumol is not limited by the production method or anything.

Xanthohumol is a component derived from hops, and can be extracted from hops with a solvent. For example, dried hops are ground into pellets which are then immersed in an organic solvent such as an alcohol for extraction. Then, the resulting liquid extract is concentrated or dried, followed by separation or purification by chromatography or the like, whereby xanthohumol can be obtained. The temperature of extraction from hops, fractionation, and purification is preferably lower than 80°C, more preferably 5°C to 70°C, for example.

Commercially available xanthohumol can also be used. In the present invention, purified xanthohumol may be used. The composition of the present invention may contain a material derived from plants rich in xanthohumol as long as the effect of the present invention can be achieved. The material derived from plants rich in xanthohumol may be a concentrate, dried matter, or the like of the liquid extract.

As indicated by examples described later, isoxanthohumol and xanthohumol exhibited an effect of promoting nitric oxide production in vascular endothelial cells. Thus, isoxanthohumol and xanthohumol have an effect of improving vascular endothelial function (vascular endothelial cellular function). Increasing the amount of nitric oxide production in vascular endothelial cells can widen blood vessels and increase the amount of blood flow (promoting blood circulation), for example. Thus, promoting the amount of nitric oxide production in vascular endothelial cells improves blood flow. Isoxanthohumol and xanthohumol can improve blood flow by the effect of promoting nitric oxide production in vascular endothelial cells. In one embodiment, isoxanthohumol and xanthohumol can be used as active ingredients for widening or relaxing blood vessels.

Isoxanthohumol and xanthohumol are compounds present in natural products, foods, and beverages and that have been used in foods and beverages. Thus, daily intake of isoxanthohumol and xanthohumol, for example, is unlikely to cause any problems in terms of safety. Thus, the present invention can provide a composition for improving blood flow and a composition for improving vascular endothelial function, each containing a highly safe component as an active ingredient. Isoxanthohumol has been also reported to be stable even at a high temperature of 100°C, for example. The Food Sanitation Act defines sterilization conditions as the standard of soft drinks and the like. For example, soft drinks having a pH of 4.0 or higher (excluding those having a pH of 4.6 or higher and a water activity higher than 0.94) need to be heated at 85°C for 30 minutes. In view of conversion of ingredients in such a sterilization process, isoxanthohumol is considered to be more highly suitable for beverage applications.

In one embodiment, the present invention can provide various functional foods, functional beverages, and the like that exhibit an effect of improving blood flow or an effect of improving vascular endothelial function, that are thermally stable, and that contribute to maintaining and improving the health.

Isoxanthohumol and xanthohumol can be quantified by, for example, high performance liquid chromatography (HPLC) or LC-MS/MS. Advantageously, isoxanthohumol and xanthohumol are easy to quantify and thus easy to use as active ingredients of foods with function claims and the like which require quantitative analysis and standardization of active ingredients.

The first composition for improving blood flow of the present invention, the first composition for improving vascular endothelial function of the present invention, the second composition for improving blood flow of the present invention, and the second composition for improving vascular endothelial function of the present invention are also collectively referred to as the composition for improving blood flow or the like of the present invention.

As described above, isoxanthohumol and xanthohumol have an effect of promoting nitric oxide production in vascular endothelial cells. Such an effect of improving vascular endothelial function can widen blood vessels and increase the amount of blood flow. The effect of improving vascular endothelial function can also maintain the health (functionality) of blood vessels and maintain blood vessel flexibility, elasticity, and the like. Thus, isoxanthohumol and xanthohumol are useful in, for example, maintaining or improving blood vessel flexibility, elasticity, and the like. In one embodiment, the first composition for improving vascular endothelial function and the second composition for improving vascular endothelial function of the present invention can be used to maintain or improve blood vessel flexibility and/or blood vessel elasticity.

The composition for improving blood flow or the like of the present invention can be used to prevent or ameliorate a symptom or disease that can be expected to be prevented or ameliorated by improving blood flow or improving vascular endothelial function. Examples of such a symptom or disease include a symptom or disease resulting from reduced blood flow.

Reduced blood flow (reduced amount of blood flow) causes symptoms such as cold sensitivity, shoulder stiffness, skin problems (poor skin conditions), swelling, and fatigue. Improving blood flow is effective in preventing or ameliorating such symptoms or diseases resulting from reduced blood flow. The composition for improving blood flow or the like of the present invention can be suitably used for, for example, preventing or reducing cold sensitivity, warm-feeling (temperature sensation), preventing or reducing shoulder stiffness, improving skin conditions, preventing or reducing swelling, recovering from fatigue, and reducing fatigue.

Herein, prevention of a symptom or disease includes preventing the onset of a symptom or disease, delaying the onset of a symptom or disease, reducing the incidence of a symptom or disease, and reducing the onset risk of a symptom or disease. Amelioration of a symptom or disease includes helping a subject recover from a symptom or disease, alleviating a symptom or disease, and delaying or preventing symptom exacerbation or disease progression.

The composition for improving blood flow or the like of the present invention is applicable for either therapeutic use (medical use) or non-therapeutic use (non-medical use). The "non-therapeutic" is a concept that does not include medical activities, i.e., a concept that does not include surgery, therapy, or diagnosis.

The composition for improving blood flow or the like of the present invention can be provided as a food or beverage, a pharmaceutical or quasi-pharmaceutical product, feed, or the like. The composition for improving blood flow or the like of the present invention may be a food or beverage, a pharmaceutical or quasi-pharmaceutical product, feed, or the like by itself for improving blood flow or improving vascular endothelial function; or may be a material, a preparation, or the like to be added to such a product or the like.

For example, the composition for improving blood flow or the like of the present invention is provided as an agent, but is not limited thereto. The agent can be provided directly as a composition, or can be provided as a composition containing the agent. In one embodiment, the composition for improving blood flow of the present invention can be regarded as a blood flow improving agent. In one embodiment, the composition for improving vascular endothelial function can be regarded as a vascular endothelial function improving agent.

Preferably, the composition for improving blood flow or the like of the present invention is a composition for oral intake in order to sufficiently obtain the effect of the present invention, but may be in any form. Examples of the composition for oral intake include foods, beverages, pharmaceutical products for oral intake, quasi-pharmaceutical products for oral intake, and feed. Preferred are foods, beverages, and pharmaceutical products for oral intake. More preferred are foods and beverages. The composition for improving blood flow or the like of the present invention may be in a form different from the composition for oral intake. Examples of the form different from the composition for oral intake include a form that is likely to allow for percutaneous or mucosal absorption of the active ingredient. A preferred example is a topical agent.

The composition for improving blood flow or the like of the present invention can contain optional additives and optional components, in addition to the active ingredient (isoxanthohumol or xanthohumol) used in the present invention, as long as the effect of the present invention is not impaired. Such additives and components can be selected according to the composition form or the like. Generally, those that can be used in foods, beverages, pharmaceutical products, quasi-pharmaceutical products, feed, and the like can be used.

When the composition for improving blood flow or the like of the present invention is provided as a food or beverage, a pharmaceutical or quasi-pharmaceutical product, feed, or the like, any common method can be used for the production. For example, the composition for improving blood flow or the like that contains isoxanthohumol can also be produced by adding a hop extract and heating the hop extract during the production.

For example, when the composition for improving blood flow or the like of the present invention is provided as a food or beverage, various types of foods or beverages can be provided by, for example, adding a component usable in a food or beverage (e.g., a food material or an optional food additive) to the active ingredient used in the present invention. Non-limiting examples of the food or beverage include general foods and beverages, health foods, health beverages, foods with function claims, foods for specified health uses, and foods and beverages for the sick. The health foods, the foods with function claims, the foods for specified health uses, and the like can be used in various forms of preparations such as fine granules, tablets, granules, powders, capsules, chewable tablets, syrups, liquids, and liquid foods.

An example of a preferred embodiment of the composition for improving blood flow or the like of the present invention may be a beverage.

The beverage may be a non-alcoholic beverage or an alcoholic beverage. Examples of the non-alcoholic beverage include tea-based beverages, coffee beverages, non-alcoholic beer-taste beverages, carbonated beverages, functional beverages, fruit and/or vegetable-based beverages, lactic beverages, soy milk beverages, and flavored water.

When the composition for improving blood flow or the like of the present invention is a beverage, preferably, it is a tea-based beverage, a coffee beverage, an alcoholic beverage, a non-alcoholic beer-taste beverage, a carbonated beverage, a functional beverage, a fruit and/or vegetable-based beverage, a lactic beverage, a soy milk beverage, or flavored water.

When the composition for improving blood flow or the like of the present invention is a tea-based beverage, preferably, it is a black tea beverage or a sugarless tea beverage. Examples of the sugarless tea beverages include green tea beverages, oolong tea beverages, barley tea beverages, brown rice tea beverages, adlay tea beverages, and sugarless black tea beverages.

When the composition for improving blood flow or the like of the present invention is a coffee beverage, preferably, it is a packaged coffee beverage or liquid coffee.

Examples of the alcoholic beverage include beer, beer-based beverages, and alcoholic beverages other than the beer and beer-based beverages.

When the composition for improving blood flow or the like of the present invention is a beer-based beverage, preferably, it is low-malt beer or a beer-like beverage.

When the composition for improving blood flow or the like of the present invention is an alcoholic beverage other than the beer and beer-based beverages, preferably, it is *shochu,* a *shochu* highball, a liqueur, cocktail, a spirit, or a whisky.

The term "non-alcoholic beer-taste beverage" as used herein refers to carbonated beverages with beer-like flavors, which are non-fermented non-alcoholic beverages, substantially free of alcohols. Here, the non-alcoholic beer-taste beverage does not exclude beverages containing a trace amount (undetectable amount) of alcohol.

When the composition for improving blood flow or the like of the present invention is a carbonated beverage, preferably, it is a cola-flavored beverage, a clear carbonated beverage, ginger ale, a fruit juice-based carbonated beverage, a milk-containing carbonated beverage, or a sugarless carbonated beverage.

When the composition for improving blood flow or the like of the present invention is a functional beverage, preferably, it is a sports drink, an energy drink, a health-supporting beverage, or a jelly drink pouch.

When the composition for improving blood flow or the like of the present invention is a fruit and/or vegetable-based beverage, preferably, it is a 100% fruit juice, a fruit-containing beverage, a soft drink with a low fruit juice content, a pulp-containing fruit juice, or a pulp-containing beverage.

When the composition for improving blood flow or the like of the present invention is a lactic beverage, preferably, it is milk, a yogurt drink, a lactic acid bacteria beverage, or a milk-containing soft drink.

When the composition for improving blood flow or the like of the present invention is a soy milk beverage, preferably, it is soy milk or a soybean beverage.

Since isoxanthohumol is thermally stable, the composition for improving blood flow or the like that contains isoxanthohumol is more suitable for a beverage.

The form of the beverage is not particularly limited. Examples include packaged beverages. Packages for the packaged beverages are not particularly limited. Packages in any form and of any material may be used. For example, any of the following commonly used packages can be used: metal packages such as aluminum cans and steel cans; resin containers such as plastic bottles; paper containers such as cartons; glass containers such as glass bottles; and wooden containers such as barrels. The beverage is filled and sealed in any of these packages, whereby a packaged beverage can be obtained.

When the composition for improving blood flow or the like of the present invention is provided as a pharmaceutical or quasi-pharmaceutical product, various dosage forms of pharmaceutical or quasi-pharmaceutical products can be provided by, for example, adding a pharmacologically acceptable carrier, an optional additive, or the like to the active ingredient used in the present invention. Such a carrier, an additive, or the like may be of any pharmacologically acceptable type that can be used in pharmaceutical or quasi-pharmaceutical products. Examples thereof include excipients, binders, disintegrants, lubricants, antioxidants, and colorants. One or more of these can be used. The form of administration (intake) of the pharmaceutical or quasi-pharmaceutical product may be oral administration or parenteral administration (percutaneous administration, transmucosal administration, enteral administration, injection, or the like). Oral administration is preferred in order to more sufficiently obtain the effect of the present invention. When the composition for improving blood flow or the like is provided as a pharmaceutical or quasi-pharmaceutical product, preferably, it is a pharmaceutical or quasi-pharmaceutical product for oral intake. Examples of the dosage form of preparations for oral administration include liquids, tablets, powders, fine granules, granules, sugar-coated tablets, capsules, suspensions, emulsions, and chewable tablets. Examples of the dosage form for parenteral administration include topical agents, injections, intravenous agents, suppositories, pernasal agents, and transpulmonary agents (inhalants). Examples of the topical agents include topical skin agents such as skin adhesive agents (e.g., patches), quasi-drug cosmetics, ointments, creams, and bath additives (which are introduced into a bathtub during bathing). The pharmaceutical product may be a pharmaceutical product for non-human animals.

Providing the composition for improving blood flow or the like of the present invention as feed only requires adding the active ingredient used in the present invention to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of the active ingredient in the composition for improving blood flow or the like of the present invention is not limited, and can be set according to the composition form or the like.

The amount of isoxanthohumol in each of the first composition for improving blood flow and the first composition for improving vascular endothelial function of the present invention is, for example, preferably 0.0001 wt% or more, more preferably 0.001 wt% or more, and is preferably 90 wt% or less in the composition. In one embodiment, the amount of isoxanthohumol in each of the composition for improving blood flow or the like is preferably 0.0001 to 90 wt%, more preferably 0.001 to 90 wt%. In one embodiment, when each of the first composition for improving blood flow and the first composition for improving vascular endothelial function of the present invention is provided as a food or beverage, a pharmaceutical or quasi-pharmaceutical product, feed, or the like, preferably, the amount of isoxanthohumol is in the above ranges.

The amount of xanthohumol in each of the second composition for improving blood flow and the second composition for improving vascular endothelial function of the present invention is, for example, preferably 0.0001 wt% or more, more preferably 0.001 wt% or more, and is preferably 90 wt% or less in the composition. In one embodiment, the amount of xanthohumol in the composition for improving blood flow or the like is preferably 0.0001 to 90 wt%, more preferably 0.001 to 90 wt%. In one embodiment, when each of the second composition for improving blood flow and the second composition for improving vascular endothelial function of the present invention is provided as a food or beverage, a pharmaceutical or quasi-pharmaceutical product, feed, or the like, preferably, the amount of xanthohumol is in the above ranges.

The intake (dosage) of the composition for improving blood flow or the like of the present invention is not limited, as long as it is the amount (effective amount) that provides an effect of improving blood flow or an effect of improving vascular endothelial function. The intake may be suitably set according to the dosage form, administration or intake method, body weight of a subject, and the like.

In one embodiment, when the first composition for improving blood flow or the first composition for improving vascular endothelial function is orally fed or administered to a human (adult), the intake of the composition in terms of intake of isoxanthohumol is preferably 1 to 200 mg, more preferably 5 to 60 mg, per day for body weight of 60 kg. Feeding or administration of isoxanthohumol in the above amount in one or more portions per day, for example, one to several portions (e.g., two to three portions) per day, is preferred.

In one embodiment, when the second composition for improving blood flow or the second composition for improving vascular endothelial function is orally fed or administered to a human (adult), the intake of the composition in terms of intake of xanthohumol is preferably 1 to 200 mg, more preferably 5 to 60 mg, per day for body weight of 60 kg. Feeding or administration of xanthohumol in the above amount in one or more portions per day, for example, one to several portions (e.g., two to three portions) per day, is preferred.

The composition for improving blood flow or the like of the present invention can be fed or administered by a method appropriate to the form. Preferably, the composition for improving blood flow or the like of the present invention is orally fed (orally administered) in order to sufficiently obtain the effect of the present invention, but may not necessarily be orally fed or administered, for example, as long as it is in a form that is likely to allow for percutaneous or mucosal absorption of the active ingredient.

A subject to be fed (administered) with the composition for improving blood flow or the like of the present invention is not limited. The subject is preferably a human or non-human mammal, more preferably a human.

Preferably, the subject to be fed or administered with the composition for improving blood flow or the like of the present invention is one who needs or wants to improve blood flow or one who needs or wants to improve vascular endothelial function. Examples of the subject include humans with symptoms such as cold sensitivity, shoulder stiffness, skin problems (poor skin conditions), swelling, or fatigue. The composition for improving blood flow or the like of the present invention can also be used, for example, for a healthy person for purposes such as prevention of a symptom or disease that can be expected to be prevented or ameliorated by blood flow improvement or vascular endothelial function improvement.

The composition for improving blood flow or the like of the present invention may be labeled with a function claim based on the effect of improving blood flow or the effect of improving vascular endothelial function. The composition for improving blood flow or the like of the present invention may be labeled with one or more of the following function claims, for example: "improving vascular endothelial function", "preventing a reduction in vascular endothelial function", "maintaining the health of blood vessels", "maintaining blood vessel flexibility", "widening blood vessels to maintain blood flow", "promoting blood circulation", "improving blood flow to maintain the body temperature", "improving blood flow to prevent or reduce cold sensitivity", "warm-feeling (temperature sensation)", "improving blood flow to prevent or reduce shoulder stiffness", "improving blood flow to promote recovery from fatigue", "reducing fatigue", "preventing or reducing swelling", and "improving blood flow to improve skin conditions".

In one embodiment of the present invention, preferably, the composition for improving blood flow or the like of the present invention is a food or beverage labeled with one or more of the above function claims. The label may be a label indicating use for obtaining these functions. The label may be directly attached to the composition or may be attached to a container or package of the composition.

The present invention also encompasses the following methods:
a method of improving blood flow, the method including administering or feeding isoxanthohumol to a subject;
a method of improving vascular endothelial function, the method including administering or feeding isoxanthohumol to a subject;
a method of improving blood flow, the method including administering or feeding xanthohumol to a subject; and
a method of improving vascular endothelial function, the method including administering or feeding xanthohumol to a subject.

Preferably, the method of improving blood flow is a method of improving blood flow by improving vascular endothelial function.

Each method described above may be a therapeutic or non-therapeutic method.

Administering or feeding isoxanthohumol or xanthohumol to a subject promotes nitric oxide production in vascular endothelial cells and thus increases the amount of nitric oxide production. Such an effect makes it possible to improve blood flow and vascular endothelial function.

The present invention also encompasses the following uses:
use of isoxanthohumol for improving blood flow;
use of isoxanthohumol for improving vascular endothelial function;
use of xanthohumol for improving blood flow by improving vascular endothelial function; and
use of xanthohumol for improving vascular endothelial function.

Preferably, the use for improving blood flow is use for improving blood flow by improving vascular endothelial function.

Preferably, the use is for a human or non-human mammal, more preferably a human. Each use described above may be therapeutic or non-therapeutic use.

In the methods and the uses, the "improving blood flow", the "improving vascular endothelial function", preferred embodiments thereof, and the like are as described above for the composition for improving blood flow or the like of the present invention.

In the methods and the uses, preferably, isoxanthohumol or xanthohumol is administered or fed in one or more portions per day, for example, one to several portions (e.g., two to three portions) per day, to a subject.

The methods and the use each only require administering or feeding isoxanthohumol or xanthohumol in an amount (effective amount) that provides the effect of improving blood flow, the effect of improving vascular endothelial function, or the like. A preferred intake of isoxanthohumol or xanthohumol, a preferred subject to be fed or administered, administration or intake method, and the like are as described above for the composition for improving blood flow or the like of the present invention described above. Isoxanthohumol or xanthohumol may be directly administered or fed, or may be administered or fed as a composition containing isoxanthohumol or xanthohumol. For example, the composition for improving blood flow or the like of the present invention described above may be administered or fed. In one embodiment, isoxanthohumol or xanthohumol can be used to prevent or ameliorate a symptom or disease that can be expected to be prevented or ameliorated by blood flow improvement or vascular endothelial function improvement. For example, isoxanthohumol or xanthohumol can be used to prevent or ameliorate a symptom or disease resulting from reduced blood flow.

Isoxanthohumol and xanthohumol can also be used to produce a food or beverage, a pharmaceutical or quasi-pharmaceutical product, feed, or the like that is used to improve blood flow. In one embodiment, the present invention also encompasses use of isoxanthohumol for producing a composition for improving blood flow; use of xanthohumol for producing a composition for improving blood flow; use of isoxanthohumol for producing a composition for improving vascular endothelial function; and use of xanthohumol for producing a composition for improving vascular endothelial function.

### EXAMPLES

The following provides examples that more specifically describe the present invention, but the scope of the present invention is not limited thereto.

### <Example 1>

### Effect of xanthohumol and isoxanthohumol in promoting nitric oxide (NO) production in vascular endothelial cells

The effect of xanthohumol and isoxanthohumol (both produced by Gifu Shellac Manufacturing Co., Ltd.) in promoting nitric oxide (NO) production in vascular endothelial cells was evaluated by the following procedure. Specifically, human umbilical vein endothelial cells (HUVECs, Lonza) were treated with xanthohumol or isoxanthohumol for 24 hours, and the amount of nitric oxide (NO) production was measured (n = 5 in each case).

### (Procedure)

HUVECs in a medium (EGM-2, Lonza) were seeded onto a 96-well plate at 10000 cells/0.1 mL/well, and cultured in a CO₂ incubator. On the following day, a xanthohumol-added group was transferred to a medium to which xanthohumol was added; an isoxanthohumol-added group was transferred to a medium to which isoxanthohumol was added; a control group was transferred to a control medium; and a positive control group was transferred to a medium containing positive control (Lovastatin, 10 µM). Then, these groups were cultured in a CO₂ incubator for additional 24 hours. The medium to which isoxanthohumol was added was prepared by dissolving isoxanthohumol in dimethyl sulfoxide (DMSO) and adding the solution to a medium such that the final concentration of isoxanthohumol was 1 µg/mL, 3 µg/mL, or 10 pg/mL. The medium to which xanthohumol was added was prepared by dissolving xanthohumol in DMSO and adding the solution to a medium such that the final concentration of xanthohumol was 1 µg/mL, 3 µg/mL, or 10 µg/mL. The control medium was prepared by adding DMSO to a medium to the same concentration as that in the other groups. The medium containing positive control was prepared by adding a solution of lovastatin (produced by FUJIFILM Wako Pure Chemical Corporation) in DMSO to a medium such that the final concentration of lovastatin was 10 µM.

After culturing for 24 hours, diaminofluorescein-2 diacetate (DAF-2, Goryo Chemical, Inc.) was added to each medium to a final concentration of 10 µM, followed by incubation in a CO₂ incubator for two hours. Subsequently, the culture supernatant was transferred to a 96-well plate (black plate), and the fluorescence intensity (ex. 495 nm, em. 515 nm) was measured. The fluorescence intensity relative to the fluorescence intensity of the control group was determined as the relative amount of NO released. Significant difference was tested between the groups (control group vs. xanthohumol-added group, or control group vs. isoxanthohumol-added group) by Student's t-test (significant level: p < 0.05). Tables 1 and 2 below each show the relative amount of NO released (%) of each group (average ± standard error).

**[Table 1]**

| | Xanthohumol concentration (µg/mL) | Relative amount of NO released (%) |
|---|---|---|
| Control group | 0 | 100.0 ± 1.8 |
| Xanthohumol-added group | 1 | 108.3 ± 2.5 |
| | 3 | 124.8 ± 3.6 |
| | 10 | 147.5 ± 7.9 |

**[Table 2]**

| | Isoxanthohumol concentration (µg/mL) | Relative amount of NO released (%) |
|---|---|---|
| Control group | 0 | 100.0 ± 1.8 |
| Isoxanthohumol -added group | 1 | 108.2 ± 2.7 |
| | 3 | 119.6 ± 2.1 |
| | 10 | 111.7 ± 3.4 |

The isoxanthohumol-added groups (isoxanthohumol concentration: 1 µg/mL, 3 µg/mL, and 10 µg/mL) and the xanthohumol-added groups (xanthohumol concentration: 1 µg/mL, 3 µg/mL, and 10 µg/mL) were all significantly different from the control group. The relative amount of NO released of the positive control group was 122.8 ± 2.7% (significantly different). The above results show that xanthohumol and isoxanthohumol have a physiological effect of increasing nitric oxide (NO) production in vascular endothelial cells (vascular endothelial function improving effect).

## Claims

1. A composition for improving blood flow, the composition comprising:
isoxanthohumol as an active ingredient.

2. The composition for improving blood flow according to claim 1,
wherein the composition improves blood flow by improving vascular endothelial function.

3. The composition for improving blood flow according to claim 2,
wherein the improving vascular endothelial function is promoting nitric oxide production in vascular endothelial cells.

4. A composition for improving vascular endothelial function, the composition comprising:
isoxanthohumol as an active ingredient.

5. A composition for improving blood flow which improves blood flow by improving vascular endothelial function, the composition comprising:
xanthohumol as an active ingredient.

6. The composition for improving blood flow according to claim 5,
wherein the improving vascular endothelial function is promoting nitric oxide production in vascular endothelial cells.

7. A composition for improving vascular endothelial function, the composition comprising:
xanthohumol as an active ingredient.

8. The composition according to any one of claims 1 to 7,
wherein the composition is a food or beverage.

9. The composition according to any one of claims 1 to 8,
wherein the composition is a beverage.

10. The composition according to claim 9,
wherein the beverage is a tea-based beverage, a coffee beverage, an alcoholic beverage, a non-alcoholic beer-taste beverage, a carbonated beverage, a functional beverage, a fruit and/or vegetable-based beverage, a lactic beverage, a soy milk beverage, or flavored water.

11. The composition according to any one of claims 1 to 10,
wherein the composition is labeled with one or more of the following function claims: "improving vascular endothelial function", "preventing a reduction in vascular endothelial function", "maintaining the health of blood vessels", "maintaining blood vessel flexibility", "widening blood vessels to maintain blood flow", "promoting blood circulation", "improving blood flow to maintain the body temperature", "improving blood flow to prevent or reduce cold sensitivity", "warm-feeling (temperature sensation)", "improving blood flow to prevent or reduce shoulder stiffness", "improving blood flow to promote recovery from fatigue", "reducing fatigue", "preventing or reducing swelling", and "improving blood flow to improve skin conditions".

12. A method of improving blood flow, the method comprising:
administering or feeding isoxanthohumol to a subject.

13. A method of improving vascular endothelial function, the method comprising:
administering or feeding isoxanthohumol to a subject.

14. A method of improving blood flow by improving vascular endothelial function, the method comprising,
administering or feeding xanthohumol to a subject.

15. A method of improving vascular endothelial function, the method comprising
administering or feeding xanthohumol to a subject.

16. Use of isoxanthohumol for improving blood flow.

17. Use of isoxanthohumol for improving vascular endothelial function.

18. Use of xanthohumol for improving blood flow by improving vascular endothelial function.

19. Use of xanthohumol for improving vascular endothelial function.
